# EUROPÄISCHE PATENTSCHRIFT

(11) **EP 0 699 205 B1**
(45) Veröffentlichungstag und Bekanntmachung des Hinweises auf die Patenterteilung: **03.09.1997**
(21) Anmeldenummer: 94916220.0
(22) Anmeldetag: 09.05.1994
(51) Int. Cl.: C07H 15/04

(54) **VERFAHREN ZUR HERSTELLUNG HELLFARBIGER ALKYL- UND/ODER ALKENYLOLIGOGLYKOSIDE**
PROCESS FOR PREPARING LIGHT-COLOURED ALKYL AND/OR ALKENYL OLIGOGLYCOSIDES
PROCEDE DE PREPARATION D'OLIGOGLYCOSIDES D'ALKYLE ET/OU D'ALCENYLE DE COULEUR CLAIRE

(30) Priorität: 18.05.1993 DE 4316601
(43) Veröffentlichungstag der Anmeldung: 06.03.1996
(73) Patentinhaber: Henkel Kommanditgesellschaft auf Aktien, 40191 Düsseldorf (DE)
(72) Erfinder: WEUTHEN, Manfred, D-42697 Solingen (DE)
(86) Internationale Anmeldenummer: EP9401484
(87) Internationale Veröffentlichungsnummer: WO9426757

(56) Entgegenhaltungen:
- EP-A- 0 338 151
- EP-A- 0 388 857
- EP-A- 0 389 753
- WO-A-92/11270
- WO-A-93/22324

## Beschreibung

Die Erfindung betrifft ein Verfahren zur Herstellung von hellfarbigen Alkyl- und/oder Alkenyloligoglykosiden, bei dem man die Neutralisation der sauren Katalysatoren in Gegenwart von Reduktionsmitteln durchführt.

### Stand der Technik

Alkyloligoglykoside und insbesondere Alkyloligoglucoside stellen nichtionische Tenside dar, die infolge ihrer ausgezeichneten Detergenseigenschaften und ihrer hohen ökotoxikologischen Verträglichkeit zunehmend an Bedeutung gewinnen. Zu ihrer Herstellung wird von Zuckern oder Stärkeabbauprodukten ausgegangen, die üblicherweise in Gegenwart saurer Katalysatoren acetalisiert werden. Aus Gründen des Massenwirkungsgesetzes empfiehlt es sich, das Kondensationswasser kontinuierlich aus dem Reaktionsgleichgewicht zu entfernen und die eine Komponente, gewöhnlich den preiswerteren Fettalkohol, in ausreichendem Überschuß einzusetzen. Nach Abschluß der Reaktion wird der saure Katalysator beispielsweise mit Natriumhydroxid und/oder Magnesiumoxid neutralisiert und der überschüssige Fettalkohol bis auf einen Restgehalt < 1 Gew.-% abdestilliert.

Die nach der Destillation anfallenden Alkyloligoglykoside sind stark verfärbt und aus ästhetischen Gründen für die Vermarktung ungeeignet. Zur Herstellung hellfarbiger Produkte ist es daher erforderlich, nach der Destillation eine Bleiche durchzuführen. Es liegt dabei auf der Hand, daß ein großes Bedürfnis nach Verfahrensweisen besteht, die die Verfärbung der rohen Reaktionsprodukte im Verlauf ihrer Herstellung wenn nicht verhindern, so doch wenigstens begrenzen. Die Aufgabe besteht folglich darin, bei gleicher Menge an Bleichmitteln hellfarbigere Produkte bereitzustellen bzw. farblich zufriedenstellende Produkte mit einer vergleichsweise geringeren Menge an Bleichmitteln zu erhalten.

Ein weiteres Problem bei der Herstellung der Alkyloligoglykoside besteht in der Qualität des Fettalkohols, der im Verlauf der Destillation rückgewonnen und in den Prozeß zurückgeführt wird. Bei kontinuierlicher Fahrweise ist eine zunehmende Verfärbung des Destillatalkohols einhergehend mit einer Anreicherung von Carbonylverbindungen zu beobachten, die eine separate Aufarbeitung erforderlich machen. Auch hier ist es wünschenswert, das Verfahren zur Herstellung von Alkyloligoglykosiden so auszugestalten, daß die Qualitätsverschlechterung wenn nicht vermieden, so doch begrenzt werden kann.

### Beschreibung der Erfindung

Gegenstand der Erfindung ist ein Verfahren zur Herstellung von Alkyl- und/oder Alkenyloligoglykosiden der Formel **(I)**,

**R**^{**1**}**O-[G]**_{**p**} **(I)**

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht, durch sauer katalysierte Acetalisierung von Zuckern mit primären Alkoholen, bei dem man die Reaktionsmischung in Gegenwart von Reduktionsmitteln neutralisiert sind aus der Gruppe, die gebildet wird von, die ausgewählt
(a) Phosphorsäuren oder deren Alkalisalzen, in denen der Phosphor eine Oxidationszahl kleiner 5 aufweist und
(b) Schwefelsäuren oder deren Alkalisazen, in denen der Schwefel eine Oxidationszahl kleiner 6 aufweist,
und anschließend in an sich bekannter Weise bleicht.

Überraschenderweise wurde gefunden, daß der Zusatz von Reduktionsmitteln in der Neutralisation der rohen Acetalisierungsprodukte nach der Bleiche zu Produkten mit signifikant verbesserter Farbe führt. Gleichzeitig werden Farbe und Carbonylzahl des nach Destillation zurückgewonnenen Fettalkohols in solcher Weise verbessert, daß eine separate Aufarbeitung auch nach einer großen Zahl von Kreisläufen unterbleiben kann.

### Alkyl - und/oder Alkenyloligoglykoside

Alkyl- und Alkenyloligoglykoside stellen bekannte Stoffe dar, die nach den einschlägigen Verfahren der präparativen organischen Chemie erhalten werden können. Stellvertretend für das umfangreiche Schrifttum sei hier auf die Schriften **EP-A1-0 301 298** und **WO 90/3977** verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside.

Die Indexzahl p in der allgemeinen Formel (I) gibt den Oligomerisierungsgrad (DP-Grad), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese anfallen. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3).

Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalko- hol, Isostearylalkohol, Oleylalkohol, Elaidylalkohol, Petroselinylalkohol, Arachylalkohol, Gadoleylalkohol, Behenylalko- hol, Erucylalkohol, sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### Reduktionsmittel

Im Zusammenhang mit Tensiden ist die Verwendung von Reduktionsmitteln zur Qualitätsverbesserung aus Einzelbeispielen bekannt, wenngleich die Dosierung in der Neutralisation neu ist. So wird beispielsweise in der **EP-B1 0 077 167** (Rohm & Haas) vorgeschlagen, die Acetalisierung von Glucose in Gegenwart eines sauren Katalysators und eines Reduktionsmittels durchzuführen. Gemäß der **EP-B1 0 165 721** (Staley) werden hellfarbige Alkyloliglykoside gewonnen, indem man die verfärbten Produkte mit einem Bleichmittel und einer SO₂-Quelle behandelt. Gegenstand der **EP-A1 0 338 151** (Henkel) ist ein Verfahren, bei dem man die Bleiche der Alkyloligoglykoside in Gegenwart von Reduktionsmitteln, beispielsweise Natriumboranat oder Raney-Nickel, durchführt. Aus der **EP-0 388 857** (Kao) ist schließlich ein Verfahren bekannt, bei man die rohen Reaktionsprodukte nach der Neutralisation der sauren Katalysatoren mit Boranaten behandelt und diese anschließend wieder durch Zugabe von Säuren zerstört. Obschon nach den genannten Verfahren zwar Produkte erhalten werden können, die farblich mehr oder minder zufriedenstellend sind, haben sie doch keine Auswirkung auf die Qualität des abzutrennenden überschüssigen Fettalkohols und sind somit im Hinblick auf die vorliegende komplexe Aufgabenstellung unbefriedigend.

Im Sinne des erfindungsgemäßen Verfahrens kommen als Reduktionsmittel beispielsweise in Betracht:
*** Phosphorsäuren oder deren Alkalisalze, in denen der Phosphor eine Oxidationszahl kleiner 5 aufweist, beispielsweise Phosphorige Säure, Unterphosphorige Säure, Natriumhypophosphit;
*** Schwefelsäuren oder deren Alkalisalze, in denen der Schwefel eine Oxidationszahl kleiner 6 aufweist, beispielsweise Schweflige Säure und Natriumdithionit.

Üblicherweise können die Reduktionsmittel in Mengen von 0,5 bis 5, vorzugsweise 1 bis 3 Gew.-% - bezogen auf die Glykose - eingesetzt werden. Aus anwendungstechnischer Sicht hat sich der Einsatz von 1 bis 3 Gew.-% Natriumhypophosphit als besonders vorteilhaft erwiesen.

### Acetalisierung

Die Herstellung der Alkyl- und/oder Alkenyloligoglykoside durch Acetalisierung von Zucker mit Fettalkoholen ist an sich bekannt. Üblicherweise werden Glucose und Fettalkohol im molaren Verhältnis 1 : 2 bis 1 : 8 vorgelegt, mit einem sauren Katalysator versetzt (beispielsweise p-Toluolsulfonsäure oder Alkylbenzolsulfonsäure) und unter kontinuierlichem Austrag des Kondensationswassers erhitzt, bis der Restglucosegehalt in der Reaktionsmischung unter 1, vorzugsweise unter 0,5 und insbesondere unter 0,05 Gew.-% liegt. Anschließend werden die Reaktionsprodukte zunächst durch Zugabe der Base - beispielsweise einem Alkali- und/oder Erdalkalioxid, -hydroxid oder -carbonat - und des Reduktionsmittels neutralisiert und schließlich der überschüssige Alkohols beispielsweise in Fallfilm- und/oder Dünnschichtverdampfern abgetrennt. Als letzte Operationen schließen sich dann die Bleiche in Gegenwart von Bleichboostern und Peroxidverbindungen sowie das Anpasten der Produkte an. Die Bedingungen und Mengenangaben, unter denen die Neutralisation und Bleiche der Alkyl- und/ oder Alkenyloligoglykoside durchgeführt wird, sind zwar nicht völlig unkritisch, können aber vom Fachmann dem genannten Stand der Technik entnommen werden, ohne hierzu erfinderisch tätig werden zu müssen.

### Gewerbliche Anwendbarkeit

Die nach dem erfindungsgemäßen Verfahren erhältlichen Alkyl- und/oder Alkenyloligoglykoside sind nach Bleiche besonders hellfarbig. Sie eignen sich daher für den Einsatz in Wasch-, Spül- und Reinigungsmitteln sowie Produkten zur Haar- und Körperpflege, in denen sie in Mengen von 1 bis 50, vorzugsweise 3 bis 35 Gew.-% - bezogen auf die Mittel - enthalten sein können.

Ein weiterer Vorteil besteht darin, daß der bei Destillation rückgewonnene Fettalkohol keine Verfärbungen und so geringe Carbonylzahlen aufweist, daß er ohne Aufarbeitung in den Prozeß zurückgeführt werden kann.

Die folgenden Beispiele sollen den Gegenstand der Erfindung näher erläutern, ohne ihn darauf einzuschränken.

### Beispiele

### Beispiel 1:

**Allgemeine Herstellung von Alkyloligoglucosiden**. In einem 2-l-Dreihalskolben mit Rührer, Tropftrichter und Wasserabscheider wurden 180 g (1 mol) Glucose und 1160 g (5,8 mol) C_{12/14}-Kokosfettalkohol (Lorol^{(R)} 1214, Fa.Henkel KGaA, Düsseldorf/FRG) vorgelegt und bei vermindertem Druck (CO,2 Pa; 20 mbar) auf 110°C erhitzt. Über den Tropftrichter wurden 0,8 g (0,028 mmol) wäßrige Sulfobernsteinsäurelösung (70 gew.-%ig) zugetropft. Das bei der Acetalisierung anfallende Reaktionswasser wurde über den Wasserabscheider kontinuierlich ausgetragen und in einer mit flüssigem Stickstoff gekühlten Falle aufgefangen. Nach 7 h wurde die Reaktion abgebrochen; in dieser Zeit waren 18 ml Reaktionswasser angefallen; der Restglucosegehalt der Reaktionsmischung betrug < 0,1 Gew.-%.

Anschließend wurde das Reaktionsprodukt - gemäß Beispiel 2 bis 4, Vergleichsbeispiele V1 und V2 - neutralisiert und der überschüssige Fettalkohol bei vermindertem Druck (1 mbar) und einer Sumpftemperatur von ca. 180°C bis auf einen Restgehalt von < 1 Gew.-% abgetrennt. Das zurückbleibende Alkyloligoglucosid wurde mit Wasser bei 80°C angepastet (Feststoffgehalt ca. 50 Gew.-%) und mit 500 ppm Mg²⁺-ionen (in Form von MgSO₄ ∗ 7H₂O) versetzt. Die Bestimmung der Farbzahlen erfolgte mit einer 5 gew.-%igen Lösung in Wasser/Isopropylalkohol (1:1) in einem Klett-Photometer (1-cm-Rundküvette).

### Beispiel 2:

Die Herstellung des Alkyloligoglucosids erfolgte gemäß Beispiel 1. Die Neutralisation wurde mit 1,2 g Natriumhydroxid in Form einer 50 gew.-%igen Lösung unter Zusatz von 3 g NaH₂PO₂ ∗ H₂O durchgeführt. Die Ergebnisse sind in Tab.1 zusammengefaßt.

### Beispiel 3:

Die Herstellung des Alkyloligoglucosids erfolgte gemäß Beispiel 1; der Restglucosegehalt betrug jedoch 0,5 Gew.-%. Die Neutralisation wurde mit 2,2 g Natriumhydroxid in Form einer 50 gew.-%igen Lösung unter Zusatz von 7 g NaH₂PO₂ ∗ H₂O durchgeführt. Die Ergebnisse sind in Tab.1 zusammengefaßt.

### Beispiel 4:

Die Herstellung des Alkyloligoglucosids erfolgte gemäß Beispiel 1; der Restglucosegehalt betrug jedoch 0,5 Gew.-%. Die Neutralisation wurde mit 2,2 g Natriumhydroxid in Form einer 50 gew.-%igen Lösung unter Zusatz von 5 g Na₂O₄S₂ durchgeführt. Die Ergebnisse sind in Tab.1 zusammengefaßt.

### Vergleichsbeispiel V1:

Die Herstellung des Alkyloligoglucosids erfolgte gemäß Beispiel 1. Die Neutralisation wurde mit 1,2 g Natriumhydroxid in Form einer 50 gew.-%igen Lösung durchgeführt. Die Ergebnisse sind in Tab.1 zusammengefaßt.

### Vergleichsbeispiel V2:

Die Herstellung des Alkyloligoglucosids erfolgte gemäß Beispiel 1; der Restglucosegehalt betrug jedoch 0,5 Gew.-%. Die Neutralisation wurde mit 2,2 g Natriumhydroxid in Form einer 50 gew.-%igen Lösung durchgeführt. Die Ergebnisse sind in Tab.1 zusammengefaßt.

**Tab. 1:**

| Versuchsergebnisse | | | | | |
|---|---|---|---|---|---|
| Bsp. | Farbzahl Paste (Klett) | | | Farbe FA | COZ FA |
| | A | B | C | | |
| 2 | 80 | 17 | - | farblos | < 0,1 |
| 3 | 90 | 22 | - | farblos | 0,1 |
| 4 | 200 | 50 | - | farblos | 0,2 |
| V1 | 500 | 90 | 28 | leicht gelb | 0,6 |
| V2 | 500 | 140 | 30 | gelb | 1,2 |
| Legende: A = Farbe ungebleichte Paste B = Farbe nach Bleiche mit 0,5 Gew.-% H₂O₂ C = Farbe nach Bleiche mit 1,5 Gew.-% H₂O₂ FA = Fettalkohol COZ = Carbonylzahl | | | | | |

## Patentansprüche

1. Verfahren zur Herstellung von Alkyl- und/oder Alkenyloligoglykosiden der Formel **(I)**,
**R**^{**1**}**O-[G]**_{**p**} **(I)**
in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht, durch sauer katalysierte Acetalisierung von Zuckern mit primären Alkoholen, bei dem man die Reaktionsmischung in Gegenwart von Reduktionsmitteln neutralisiert, die ausgewählt sind aus der Gruppe, die gebildet wird von
(a) Phosphorsäuren oder deren Alkalisalzen, in denen der Phosphor eine Oxidationszahl kleiner 5 aufweist und
(b) Schwefelsäuren oder deren Alkalisalzen, in denen der Schwefel eine Oxidationszahl kleiner 6 aufweist,
und anschließend in an sich bekannter Weise bleicht.

2. Verfahren nach Anspruch 1, **dadurch gekennzeichnet**, daß man Alkyloligoglucoside der Formel (I) einsetz, in der R¹ für Alkylreste mit 4 bis 11 Kohlenstoffatornen, G für einen Glucoserest und p für Zahlen von 1 bis 10 steht.

3. Verfahren nach Anspruch 1, **dadurch gekennzeichnet,** daß man Alkyloligoglucoside der Formel (I) einsetzt, in der R¹ für Alkylreste mit 12 bis 22 Kohlenstoffatomen, G für einen Glucoserest und p für Zahlen von 1 bis 10 steht

4. Verfahren nach den Ansprüchen 1 bis 3, **dadurch gekennzeichnet,** daß man die Reduktionsmittel in Mengen von 0,5 bis 5 Gew.-% - bezogen auf die Glykose - einsetzt.

## Claims

1. A process for the production of alkyl and/or alkenyl oligoglycosides corresponding to formula **(I)**:
**R**^{**1**}**O-[G]**_{**p**} **(I)**
in which R¹ is an alkyl and/or alkenyl radical containing 4 to 22 carbon atoms, G is a sugar unit containing 5 or 6 carbon atoms and p is a number of 1 to 10,
by acid-catalyzed acetalization of sugars with primary alcohols, in which the reaction mixture is neutralized in the presence of reducing agents selected from the group consisting of
(a) acids of phosphorus or alkali metal salts thereof in which the phosphorus has an oxidation number below 5 and
(b) acids of sulfur or alkali metal salts thereof in which the sulfur has an oxidation number below 6
and is then bleached in known manner.

2. A process as claimed in claim 1, **characterized in that** alkyl oligoglucosides of formula **(I)**, in which R¹ represents C₄₋₁₁ alkyl radicals, G is a glucose unit and p is a number of 1 to 10, are used.

3. A process as claimed in claim 1, **characterized in that** alkyl oligoglucosides of formula **(I)**, in which R¹ is a C₁₂₋₂₂ alkyl radical, G is a glucose unit and p is a number of 1 to 10, are used.

4. A process as claimed in claims 1 to 3, **characterized in that** the reducing agents are used in quantities of 0.5 to 5% by weight, based on the glycose.

## Revendications

1. Procédé de préparation d'alkyl- et/ou d'alcényloligoglycosides de formule I,
R¹O[G]ₚ (I)
dans la quelle
R¹ représente un radical alkyle et/ou alcényle ayant de 4 à 22 atomes de carbone,
G représente un radical de sucre ayant 5 ou 6 atomes de carbone et,
p représente des nombres allant de 1 à 10,
par acétalisation catalysée par un acide, de sucres avec des alcools primaires, dans lequel on neutralise le mélange réactionnel en présence d'agents de réduction qui sont choisis dans le groupe qui est formé de :
a) des acides phosphoriques ou leurs sels de métal alcalin, dans lesquels le phosphore possède un indice d'oxydation plus petit que 5 et,
b) des acides sulfuriques ou leurs sels de métal alcalin dans lesquels le soufre possède un indice d'oxydation plus petit que 6 et ensuite blanchit d'une manière connue en soi.

2. Procédé selon la revendication 1,
caractérisé en ce qu'
on met en oeuvre des alkyloligoglucosides de formule (I) dans laquelle R¹ représente des radicaux alcoyle ayant de 4 à 11 atomes de carbone, G représente un reste de glucose et p représente des nombres allant de 1 à 10.

3. Procédé selon la revendication 1,
caractérisé en ce qu'
on met en oeuvre des alkyloliglucosides de formule (I) dans laquelle R¹ représente des radicaux alkyle ayant de 12 à 22 atomes de carbone, G représente un reste de glucose et p représente des nombres allant de 1 à 10.

4. Procédé selon les revendications 1 à 3,
caractérisé en ce qu'
on met en oeuvre les agents de réduction en quantités allant de 0,5 à 5 % en poids, rapporté au glycose.
